# EUROPÄISCHE PATENTANMELDUNG

(11) **EP 0 812 590 A2**
(43) Veröffentlichungstag der Anmeldung: **17.12.1997**
(21) Anmeldenummer: 97112344.3
(22) Anmeldetag: 08.11.1990
(51) Int. Cl.: A61K 31/385, A61K 47/10, A61K 47/18, A61K 47/16, A61K 47/22, A61K 47/08

(54) **Verwendung von R-Alpha-Liponsäure zur Zytoprotektion**

(30) Priorität: 09.11.1989 DE 3937323
(62) Teilanmeldung aus: 90121340.5
(71) Anmelder: ASTA Medica Aktiengesellschaft, D-01277 Dresden (DE)
(72) Erfinder: Ulrich, Heinz, Dr., 63843 Niedernberg (DE); Weischer, Carl-Heinrich, Dr., 52115 Bonn (DE); Engel, Jürgen, Prof., 63755 Alzenau (DE); Hettche, Helmut, Dr., 63128 Dietzenbach (DE)

(57) **Zusammenfassung**

R-α-Liponsäure und deren pharmazeutisch verwendbaren Salze werden zur Herstellung eines Arzneimittels zur Zytoprotektion verwendet.

## Beschreibung

### α-Liponsäure ist 1,2-Dithiacyclopentan-3-valeriansäure

α-Liponsäure ist in Form des R-Enantiomeren in Pflanzen und Tieren weit verbreitet; sie wirkt in vielen enzymatischen Reaktionen als Coenzym, stellt einen Wachstumsfaktor für manche Bakterien und Protozoen dar und wird bei Knollenblätterpilzvergiftungen eingesetzt. Weiterhin weist das α-Liponsäure-Racemat antiphlogistische, antinociceptive (analgetische) sowie zytoprotektive Eigenschaften auf.

Es wurde nun gefunden, daß bei den reinen optischen Isomeren der α-Liponsäure (R- und S-Form, das heißt R-α-Liponsäure und S-α-Liponsäure) im Gegensatz zu dem Racemat überraschend das R-Enantiomere vorwiegend antiphlogistisch und das S-Enantiomere vorwiegend antinociceptiv wirksam ist, wobei ebenfalls überraschend die antiphlogistische Wirkung des R-Enantiomeren beispielsweise um einen Faktor 10 stärker ist als diejenige des Racemats. Die antinociceptive (analgetische) Wirkung des S-Enantiomeren ist beispielsweise um einen Faktor 5 bis 6 stärker als diejenige des Racemats. Die Enantiomeren stellen daher im Vergleich zu dem Racemat sehr viel spezifischere und stärker wirksame Wirkstoffe dar.
Im Vergleich zu der α-Liponsäure, das heißt dem Racemat, bestehen insbesondere folgende Unterschiede:

Das R-Enantiomer ist vornehmlich antiphlogistisch und das S-Enantiomer vornehmlich analgetisch wirksam, wobei die optischen Isomere der α-Liponsäure hinsichtlich dieser Wirkungen jeweils um ein vielfaches (zum Beispiel mindestens den Faktor 5) stärker wirksam sind als das Racemat der α-Liponsäure.

Aufgabe der Erfindung ist daher die Bereitstellung von verbesserten Arzneimitteln mit insbesondere analgetischer und antiphlogistischer Wirkung.

Die Erfindung betrifft Arzneimittel, die als Wirkstoff entweder R-α-Liponsäure oder S-α-Liponsäure (d.h. die optischen Isomere der α-Liponsäure) oder ein pharmazeutisch verwendbares Salz dieser optischen Isomere der α-Liponsäure enthalten, deren Herstellung sowie die Verwendung der optischen Isomere der α-Liponsäure oder deren Salze zur Herstellung von entsprechenden Arzneimitteln. Diese sind insbesondere für die Bekämpfung von Schmerzzuständen und Entzündungserkrankungen geeignet. Eine zytoprotektive Wirkung ist ebenfalls vorhanden.

Die in den Patentansprüchen angegebenen Gewichtsmengen beziehen sich jeweils auf die reinen optischen Isomere der α-Liponsäure, das heißt nicht auf die Salze. Bei Verwendung von Salzen müssen die jeweils in Frage kommenden Dosismengen jeweils den Mengen der freien Säure entsprechen und dem veränderten Molgewicht entsprechend erhöht werden.

Vorzugsweise werden die optischen Isomere der α-Liponsäure, das heißt R-α-Liponsäure und S-α-Liponsäure als freie Säuren verwendet. In wässrigen Lösungen werden vorzugsweise die Salze mit pharmazeutisch verwendbaren Salzbildnern verwendet.

Die Herstellung der R-α-Liponsäure und der S-α-Lipon-säure sowie deren Salze erfolgt in bekannter Weise, beziehungsweise analog hierzu.

Als Salzbildner für die R-α-Liponsäure beziehungsweise S-α-Liponsäure kommen zum Beispiel übliche Basen beziehungsweise Kationen in Frage, die in der Salzform physiologisch verträglich sind. Beispiele hierfür sind: Alkali- oder Erdalkalimetalle, Ammoniumhydroxid, basische Aminosäuren wie Arginin und Lysin, Amine der Formel NR₁R₂R₃ worin die Reste R₁, R₂ und R₃ gleich oder verschieden sind und Wasserstoff, C₁-C₄-Alkyl oder C₁-C₄ Oxyalkyl bedeuten wie Mono und Diethanolamin, 1-Amino-2-propanol, 3-Amino-1-propanol; Alkylendiamine mit einer Alkylenkette aus 2 bis 6-C-Atomen wie Ethylendiamin oder Hexamethylentetramin, gesättigte cyclische Aminoverbindungen mit 4 - 6 Ringkohlenstoffatomen wie Piperidin, Piperazin, Pyrrolidin, Morpholin; N-Methylglucamin, Kreatin, Trometamol.

Das S-Enantiomer (S-α-Liponsäure) zeigt beispielsweise im Essigsäure-Writhing-Schmerztest an der Maus und im Randall-Selitto-Entzündungsschmerztest an der Ratte eine analgetische Wirkung, die derjenigen der α-Liponsäure (das heißt dem Racemat) um mindestens einen Faktor 5 beziehungsweise 6 überlegen ist (perorale Applikation).
So wird beispielsweise in dem vorstehend angegebenen Essigsäure-Writhing-Test eine analgetisch wirksame ED50 der S-α-Liponsäure von 10.2 mg/kg per os ermittelt (ED50 des Racemats 51,3 mg/kg per os). In dem vorstehend angegebenen Randall-Selitto-Test ergibt sich eine analgetisch wirksame ED50 der S-α-Liponsäure von 7,5 mg/kg per os (ED50 des Racemats 45,9 mg/kg).

Das R-Enantiomer (R-α-Liponsäure) zeigt beispielsweise im Carrageenin-Ödem an der Ratte eine antiphlogistische Wirkung, die derjenigen der α-Liponsäure um mindestens den Faktor 10 überlegen ist (perorale Applikation).

In dem vorstehend angegebenen Carrageenin-Ödem-Test wird zum Beispiel eine antiphlogistisch wirksame ED50 des R-Enantiomers von 4,9 mg/kg per os ermittelt (ED50 des Racemats 49,7 mg/kg).
Die niedrigste, bereits analgetisch wirksame Dosis in dem Randall-Selitto-Schmerztest für die S-α-Liponsäure ist beispielsweise 1 mg/kg per os.
Die niedrigste, bereits antiphlogistisch wirksame Dosis für die R-α-Liponsäure in dem Carrageenin-Ödem-Test ist beispielsweise 1 mg/kg per os.

Ebenso ist eine zytoprotektive Wirkung im Tierversuch sowohl für die R- und S-Form bereits ab einer Dosis von 10 mg/kg per os vorhanden.

Darüberhinaus besitzen R- und S-α-Liponsäure überraschenderweise eine wachstumshemmende Wirkung gegen Retroviren, insbesondere das Human-Immundefizienzvirus HIV (HIV-1, HIV-2) und sind daher auch geeignet zur Behandlung von Erkrankungen, die durch solche Viren verursacht werden.

So besitzen sie eine gute, wachstumshemmende Wirkung auf HIV (Typ 1 und 2), welche sich in vitro beispielsweise durch folgende virologischzellbiologische Testverfahren zeigen läßt:
1. Plaque - Reduktionstest
2. CPE - Reduktionstest
3. Bestimmung der Reversen Transkriptase im Kulturüberstand
4. Bestimmung des p24 Antigens im Kulturüberstand

So wird beispielsweise bei einer einmaligen Gabe von 0,035 mg/ml die Anzahl der infektiösen Viren (zum Beispiel HIV-1), im Zellkulturüberstand von 100 % in der positiv Kontrolle auf 0 % gesenkt. Ein virusinhibierender Effekt kann in diesem Testverfahren bereits in sehr niedrigen Dosen, beispielsweise 0,001 mg/ml nachgewiesen werden.

Als allgemeiner Dosisbereich für die Wirkung (Versuch wie oben) kommt beispielsweise in Frage: 0,0035 - 0,091 mg/ml, insbesondere 0,035 - 0,070 mg/ml.

Für die in vitro Versuche wird der Wirkstoff zum Beispiel in Benzylalkohol als Lösungsmittel eingesetzt.

Für die in vitro Untersuchungen des Replikationsverhaltens von Retroviren, insbesondere HIV, können zum Beispiel folgende Substrate eingesetzt werden:
1. Virushaltiges RPMI 1640 Medium, zum Beispiel 1X Flüssig 041-01875 (synthetisches Kulturmedium von Gibeo gemäß Moore, Gerner und Franklin, H.A. (1967), J.A.M.A. 199; 519) mit einer Konzentration von 2 × 10³ - 1 × 10⁴ infektiösen Einheiten (PFU)/ml
2. Die Zellinien Jurkat Clone E6-1, Sup T1 und HeLa CT4.

Die pharmazeutischen Zubereitungen enthalten im allgemeinen zwischen 50 mg bis 3 g als Einzeldosis, vorzugsweise 100 mg bis 1 g R- beziehungsweise S-α-Liponsäure. Die erreichten Wirkspiegel/kg Körpergewicht sollen zwischen 3,5 und 200 mg, vorzugsweise zwischen 7 und 100 mg, besonders zwischen 35 und 70 mg/kg Körpergewicht liegen. Der Wirkstoff soll aus den Zubereitungen langsam abgegeben werden.

Die Verabreichung kann beispielsweise in Form von Tabletten, Kapseln, Pillen, Dragees, Aerosolen oder in flüssiger Form erfolgen.

Als flüssige Anwendungsformen kommen zum Beispiel in Frage: alkoholische beziehungsweise wässrige Lösungen sowie Suspensionen und Emulsionen.

Bevorzugte Anwendungsformen sind zum Beispiel Tabletten, die zwischen 100 mg und 2 g oder Lösungen, die zwischen 10 mg bis 0,2g/ml Flüssigkeit aktive Substanz enthalten.

Die Einzeldosis an Wirkstoff der Formel I kann beispielsweise liegen:
a) bei oraler Arzneiform zwischen 100 mg - 3 g, vorzugsweise 200 mg - 1 g.
b) bei parenteralen Arzneiformen (zum Beispiel intravenös, intramuskulär) zwischen 100 mg - 12 g, vorzugsweise 200 mg - 6 g.
c) bei Arzneiformen zur Inhalation (Lösungen oder Aerosole) zwischen 100 mg - 2 g, vorzugsweise 200 mg -1 g.

Die Dosen gemäß a) bis c) können beispielsweise 1- bis 6mal, vorzugsweise 1- bis 4mal täglich oder aber als Dauerinfusion, beispielsweise mit Hilfe eines Infusoniaten* verabreicht werden.
Die tägliche Dosis an R- beziehungsweise S-α-Liponsäure beim Menschen soll zum Beispiel 70-80 mg pro kg Gewicht liegen; die Einzeldosis zum Beispiel bei 16-20 mg pro kg Gewicht, wobei diese Dosis zweckmäßig 4mal verabreicht wird pro Tag. Vorzugsweise beträgt die Tagesdosis 4-6 g: Die Arzneimittel enthalten daher vorzugsweise 1-1,5 g an R- beziehungsweise S-α-Lipon-säure in einer galenischen Formulierung, wobei eine solche Dosis vorzugsweise 4mal verabreicht wird.
*) Infusionsgerät zur genauen stündlichen Dosierung einer gelösten Wirksubstanz

Für die Behandlung können zum Beispiel 3 mal täglich 1 bis 4 Tabletten mit einem Gehalt von 50 mg bis 2 g wirksamer Substanz oder beispielsweise bei intravenösen Injektion 1 bis 4 mal täglich eine Ampulle/Infusionsflasche von 1 bis 500 ml Inhalt mit 200 mg bis 6 g Wirksubstanz empfohlen werden. Bei oraler Verabreichung ist die minimale tägliche Dosis beispielsweise 300 mg; die maximale tägliche Dosis bei oraler Verabreichung soll 12 g nicht überschreiten.

Die Arzneimittel können in der Humanmedizin allein oder im Gemisch mit anderen pharmakologisch aktiven Stoffen verwendet werden. Die Wirkstoffe R-beziehungsweise S-α-Liponsäure können auch mit jedem anderem gegen Retroviren insbesondere HIV wirksamen Mittel kombiniert werden, beispielsweise mit Didesoxyinosin, Didesoxycytidin, insbesondere aber mit α-Interferon und/oder Azidothymidin (AZT).
- Die angegebenen Dosismengen beziehen sich stets auf die freien Säuren R- beziehungsweise S-α-Liponsäure. Falls diese in Form ihrer Salze verwendet werden, sind die angegebenen Dosierungen/Dosierungsbereiche dem höheren Mol-Gewicht entsprechend zu erhöhen. -

Bei Kombinationen mit anderen antiretroviral-wirksamen Substanzen (Komponente b) können als Komponente b nur eine, aber auch 2 und mehr (vorzugsweise 2) antiretroviral wirksame Substanzen verwendet werden, wobei im letzteren Falle auch hier die hierfür angegebenen Dosierungen stets für die Summe der jeweils vorliegenden antiretroviralwirksamen Stoffe gelten.
Der Ausdruck "Dosierungseinheit" bezieht sich stets auf eine Einzeldosis, die pro Tag auch mehrmals verabreicht werden kann.
Falls die Dosis in Form von Enzymeinheiten angegeben wird, handelt es sich um die Dosis, die für einen ganzen Tag gilt, wobei eine solche Dosis auf einmal, vorzugsweise jedoch verteilt über einen Tag (zum Beispiel in Form von Infusion) gegeben wird. Die Dosis-Angabe in Enzymeinheiten gilt insbesondere für α-Interferon.

Für die Kombination von R- beziehungsweise S-α-Liponsäure mit der Komponente b zum Beispiel AZT, können die beiden Komponenten jeweils zum Beispiel in einem Verhältnis von 1 zu 100 bis 100 zu 1 äquimolaren Anteilen wirksamer Substanz gemischt werden, insbesondere in einem Verhältnis von 1 zu 10 bis 10 zu 1, vorzugsweise in einem Verhältnis von 1 zu 3 bis 3 zu 1 Anteilen.
Im Falle einer Kombination von R- beziehungsweise S-α-Liponsäure und α-Interferon können die beiden Komponenten zum Beispiel in folgendem Verhältnis vorliegen: 50 mg - 6 g R- beziehungsweise S-α-Liponsäure (Komponente a) zu 8 × 10⁶ Enzymeinheiten bis 1 × 10⁵ Enzymeinheiten α-Interferon, insbesondere 0,5 - 3 g Komponente a zu 1-4 × 10⁶ Enzymeinheiten α-Interferon.

In der Kombination aus R- beziehungsweise S-α-Liponsäure und anderen Komponenten gemäß b) können beide Komponenten als Gemisch vorliegen. Im allgemeinen liegen die Komponenten jedoch voneinander getrennt in einer galenischen Formulierung vor, wobei hier die hierfür bekannten galenischen Formulierungen in Frage kommen: zum Beispiel eine Komponente als Tablette oder lackierte Tablette, die andere Komponente als Pulver, beide in einer Kapsel und umgekehrt; eine Komponente in Form von Pellets, die andere als Pulver, Dragee oder Tablette und umgekehrt und wobei die beiden Formen zum Beispiel in einer Kapsel vorliegen; oder in Form von Mehrschichten- oder Manteltabletten. Es wird hierzu zum Beispiel auf das Buch von Karl Thoma, Arzneimittelstabilität, Frankfurt 1978, zum Beispiel Seite 207ff. verwiesen.

Die erfindungsgemäße Kombination kann aber auch als ein Erzeugnis vorliegen, bei dem jeweils die beiden Einzelwirkstoffe in vollständig voneinander getrennten Formulierungen vorliegen, wobei insbesondere die Komponente b, aber auch beide Komponenten (a und b) in Ampullen und/oder Infusionsflaschen enthalten sind, so daß auch eine getrennte oder auch zeitlich abgestufte Verabreichung möglich ist.

Falls solche vollständig getrennten Formulierungen vorliegen, sind diese aufeinander abgestimmt und enthalten die jeweiligen Wirkstoffe in der Dosierungseinheit in denselben Mengen und entsprechenden Gewichtsverhältnissen, in denen sie in der kombinierten Mischung vorliegen können.

Bei einem Erzeugnis für die getrennte Anwendung ist es auch möglich, daß beide Kombinationspartner nicht gleichzeitig verabreicht werden. In solchen Fällen kann zum Beispiel R- beziehungsweise S-α-Liponsäure als Dauerinfusion gegeben werden (Dosis zum Beispiel 2 - 5 g pro Tag) und die andere Komponente b gleichzeitig (Dosis zum Beispiel 50 - 800 mg oder 1-8 × 10⁶ Enzymeinheiten, vorzugsweise intramuskulär) oder auch als Dauerinfusion pro Tag oder R-beziehungsweise S-α-Liponsäure kann zum Beispiel 4mal pro Tag gegeben werden (Einzeldosis zum Beispiel 0,5 -2 g) und die andere Komponente b gleichzeitig (Dosis zum Beispiel 50 - 200 mg beziehungsweise 0,5-3 × 10⁶ Enzymeinheiten). Es können dann zum Beispiel 1 bis 3 weitere Dosen an Komponente b (zum Beispiel zwischen 50 - 200 mg beziehungsweise 0,5-3 × 10⁶ Enzymeinheiten) im Abstand von jeweils 6 und/oder 12 Stunden folgen.

Die erfindungsgemäßen Zubereitungen/Erzeugnisse können vorzugsweise auch zusätzliche Vitamine enthalten, insbesondere Vitamin B₁ und/oder Vitamin E.

Zur Behandlung von Erkrankungen, die durch Retroviren, insbesondere HIV-Viren verursacht sind, sollen entsprechende Arzneimittel eine solche Menge an R-beziehungsweise S-Liponsäure enthalten beziehungsweise in einer solchen Menge verabreicht werden, daß bei ein- oder mehrmaliger Applikation im Körper ein Wirkspiegel zwischen 3,5 und 200 mg/kg, vorzugsweise zwischen 7 und 100 mg, insbesondere zwischen 35 und 70 mg/kg Körpergewicht vorliegt.

Als allgemeiner Dosisbereich der S-α-Liponsäure für die analgetische Wirkung kommt beispielsweise in Frage:
1 - 100 mg/kg oral

Als allgemeiner Dosisbereich der R-α-Liponsäure für die antiphlogistische und zytoprotektive Wirkung kommt beispielsweise in Frage:
1 - 100 mg/kg oral.

Die S-α-Liponsäure besitzt außer der antinociceptiven (analgetischen) Hauptwirkung auch noch eine antiphlogistische und zytoprotektive Wirkung, jedoch in geringerem Maße.

Ebenso besitzt die R-α-Liponsäure außer der antiphlogistischen bzw. antiarthrotischen Hauptwirkung noch eine antinociceptive und zytoprotektive Wirkung, jedoch in geringerem Maße.

Die optischen Isomere der α-Liponsäure zeigen beispielsweise an folgenden Untersuchungsmodellen eine gute analgetische, antiphlogistische, antiarthrotische und zytoprotektive Wirkung:
MgSo₄-Writhing-Test an der Maus nach GYIRES et al. (Arch.int.pharmacodyn.therap. 267, 131-140 (1984))
Adjuvans-Arthritis an der Ratte nach NEWBOULD (Brit.J.Pharmacol. 21, 127-136 (1963))
TPA- beziehungsweise Arachidonsäure-induziertes Mäuseohrödem nach YOUNG et al. (J.Invest.Dermatol. 80, 48-52 (1983))
Na-Monojodazetat-induzierte Arthrose an Ratten beziehungsweise an Hühnern nach KALBHEN in: Arthrosis deformans, Eular-Verlag, Basel/Schweiz, 1982
TPA-induzierte Arthrose an Ratten nach WEISCHER (Agents and Actions 23, 1/2 (1988))
Intestinale Ulzerationen an Ratten nach DEL SOLDATO (Agents and Actions 16, 393-396 (1985))
Colitis-Modell an der Ratte nach WEISCHER et al (Agents and Actions , Band 26, 1/2, Seite 222 bis 223, (1989))
Ethanol-Ulkus-Modell an der Ratte (Nachweis für beispielsweise eine zytoprotektive Wirkung).

Die optischen Isomere der α-Liponsäure hemmen beispielsweise die akute Entzündung sowie den Entzündungsschmerz, und sie besitzen eine spezifische zytoprotektive Wirkung.

Als Indikation kommen beispielsweise in Betracht:
Entzündliche, degenerative artikuläre und extraartikuläre rheumatische Erkrankungen, nicht-rheumatische Entzündungs- und Schwellungszustände, Arthrosis deformans, Chondropathien, Periarthritiden, entzündliche und nichtentzündliche Erkrankungen der Haut, wie zum Beispiel Neurodermitis und Psoriasis, entzündliche und nicht-entzündliche Erkrankungen des Gastrointestinaltraktes, wie zum Beispiel Gastritis, Ulcus ventriculi, Ileitis, Duodenitis, Jejunitis, Colitis, Polyneuropathie diabetogener, alkoholischer hepatischer und urämischer Genese, Leberparenchymdegeneration, Hepatitis, Fettleber und Fettzirrhose sowie chronische Lebererkrankungen, entzündliche Atemwegserkrankungen, wie zum Beispiel Asthma bronchiale, Sarkoidose, ARDS (acute respiratory distress syndrome).

Die Tagesdosen der erfindungsgemäßen Darreichungsformen für die analgetische beziehungsweise zytoprotektive beziehungsweise antiphlogistische Wirkung bestehen zum Beispiel aus 0,1 bis 600 mg, vorzugsweise 15 bis 400 mg und insbesondere 50 bis 200 mg R-α-Liponsäure oder S-α-Liponsäure.

Erfindungsgemäß wird eine Tagesdosis der optischen Isomere der α-Liponsäure (R- beziehungsweise S-Form jeweils) von 10 - 600 mg, beispielsweise von 25 bis 400 mg oder 10 bis 200 mg verabreicht. Die maximale Tagesdosis für die zytoprotektive Wirkung sowie für die Behandlung von Schmerz- und Entzündungszuständen soll 600 mg nicht überschreiten. Die Tagesdosen können in Form einer einmaligen Verabreichung der gesamten Menge oder in Form von 1 bis 6, insbesondere 1 - 4, Teildosen pro Tag eingesetzt werden. Im allgemeinen ist eine Verabreichung von 1- 4mal, insbesondere 1-bis 3mal täglich, bevorzugt.

Beispielsweise beträgt die bevorzugte Tagesdosis sowohl für die R-α-Liponsäure als auch für die S-α-Liponsäure vorzugsweise 80 mg für die parenterale Applikationsform und 200 mg für die orale Form. Insbesondere beträgt die Tagesdosis für die parenterale Applikationsform 50 mg und 150 mg für die orale Form.

Vorzugsweise werden die Arzneimittel oral verabreicht.

Die R-α-Liponsäure und die S-α-Liponsäure können insbesondere auch in Form einer Lösung appliziert werden, beispielsweise peroral, topisch, parenteral (intravenös, intraartikulär, intramuskulär, subkutan), inhalativ, transdermal.

Die Arzneimittel, die als Wirkstoff die R-α-Liponsäure oder die S-α-Liponsäure enthalten, können zum Beispiel in Form von Tabletten, Kapseln, Pillen oder Dragees, Granulaten, Pellets, Pflaster, Lösungen oder Emulsionen formuliert werden, wobei der Wirkstoff jeweils gegebenenfalls mit entsprechenden Hilfs- und Trägerstoffen kombiniert wird. Im Falle von Lösungen enthalten diese beispielsweise 0,5 bis 20 Gewichts%, vorzugsweise 1 bis 10 Gewichts% eines der optischen Isomere der α-Liponsäure (jeweils R-Form oder S-Form).

Die Dosierungseinheit der Arzneimittel mit den optischen Isomeren der α-Liponsäure oder einem therapeutisch verwendbaren Salz derselben (jeweils R-Form oder S-Form) kann beispielsweise enthalten:
a.) bei oralen Arzneiformen:
   10 bis 600 mg, vorzugsweise 20 bis 400 mg, insbesondere 50 bis 200 mg der optischen Isomere der α-Liponsäure. Die Dosen können beispielsweise 1-bis 6mal, vorzugsweise 1- bis 4mal, insbesondere 1- bis 3mal täglich verabreicht werden. Jedoch soll eine Gesamtdosis von 600 mg pro Tag für die zytoprotektive Wirkung sowie für die Behandlung von Schmerz- und Entzündungszuständen nicht überschritten werden. Dasselbe gilt auch für die folgenden unter b) bis e) aufgeführten Arzneiformen.
b.) bei parenteralen Arzneiformen (zum Beispiel intravenös, intramuskulär oder intraartikulär):
   10 bis 300 mg, vorzugsweise 15 bis 200 mg, insbesondere 20 bis 100 mg der optischen Isomere der α-Liponsäure.
   Die Dosen können beispielsweise 1- bis 6mal, vorzugsweise 1- bis 4mal, insbesondere 1- bis 3mal täglich verabreicht werden.
c.) bei Arzneiformen zur Applikation auf die Haut und Schleimhäute (zum Beispiel als Lösungen, Lotionen, Emulsionen, Salben, Pflaster und so weiter):
   10 bis 500 mg R-α-Liponsäure oder S-α-Liponsäure, vorzugsweise 40 bis 250 mg, insbesondere 50 bis 200 mg. Diese Dosen können beispielsweise 1- bis 6mal, vorzugsweise 1- bis 4mal, insbesondere 1-bis 3mal täglich verabreicht werden.
d.) bei Arzneiformen zur Inhalation (Lösungen oder Aerosole):
   0,1 bis 300 mg, vorzugsweise 0,25 bis 150 mg, insbesondere 0,5 bis 80 mg R-α-Liponsäure oder S-α-Liponsäure.
   Diese Dosen können beispielsweise 1- bis 6mal, vorzugsweise 1- bis 4mal, insbesondere 1- bis 3mal täglich verabreicht werden.
   Falls Lösungen verwendet werden, werden die optischen Isomere der α-Liponsäure vorzugsweise in Form eines Salzes eingesetzt.

Selbstverständlich können auch galenische Zubereitungen hergestellt werden, welche die oben angegebenen Dosierungseinheiten 2- bis beispielsweise 6mal enthalten. Insbesondere enthalten Tabletten oder Kapseln 20 bis 500 mg, Pellets, Pulver oder Granulate 20 bis 400 mg, Suppositorien 20 bis 300 mg R-α-Liponsäure oder S-α-Liponsäure.

Für die Bekämpfung von Retroviren (zum Beispiel AIDS) liegt die Tagesdosis zum Beispiel bei 4-6 g. Entsprechende Arzneimittel enthalten daher vorzugsweise R-α-Liponsäure oder S-α-Liponsäure in der Einzeldosis (Dosierungseinheit) beispielsweise in einer Menge von 600 mg bis 1,5 g.

Die vorstehend angegebenen Dosierungen beziehen sich stets auf die freien optischen Isomere der α-Liponsäure. Falls die optischen Isomere der α-Liponsäure in Form eines Salzes verwendet werden, sind die angegebenen Dosierungen/Dosierungsbereiche infolge des höheren Mol-Gewichtes entsprechend zu erhöhen.

Die akute Toxizität der R-α-Liponsäure beziehungsweise der S-α-Liponsäure an der Maus (ausgedrückt als LD50 mg/kg; Methode LITCHFIELD und WILCOXON, J. Pharmacol. Exp Ther. 95, 99 (1949) ), liegt beispielsweise oberhalb von 100 mg/kg bei oraler Applikation.

Für den Fall, daß die optischen Isomere der α-Liponsäure bei Tieren eingesetzt wird, kommen insbesondere folgende Indikationen in Frage: Hepatosen, Arthrosis deformans, Arthritiden und Dermatitiden.

Für die Behandlung von Tieren kommen zum Beispiel folgende Dosierungen (sowohl R-Form als auch S-Form) in Frage:

Für die Behandlung von Katzen liegt die orale Einzeldosis im allgemeinen zwischen ungefähr 2 mg/kg und 50 mg/kg Körpergewicht, die parenterale Dosis ungefähr zwischen 0,5 und 40 mg/kg Körpergewicht.

Für die Behandlung von Arthrosen bei Pferden und Vieh liegt die orale Einzeldosis im allgemeinen zwischen ungefähr 2 mg/kg und 100 mg/kg Körpergewicht, die parenterale Dosis ungefähr zwischen 0,5 und 50 mg/kg Körpergewicht.

Die optischen Isomere der α-Liponsäure sind zur Herstellung pharmazeutischer Zusammensetzungen und Zubereitungen geeignet. Die pharmazeutischen Zusammensetzungen beziehungsweise Arzneimittel enthalten die optischen Isomere der α-Liponsäure als Wirkstoff, gegebenenfalls in Mischung mit anderen pharmakologisch beziehungsweise pharmazeutisch wirksamen Stoffen. Die Herstellung der Arzneimittel erfolgt in bekannter Weise, wobei die bekannten und üblichen pharmazeutischen Hilfsstoffe sowie sonstige übliche Träger- und Verdünnungsmittel verwendet werden können. Als derartige Träger- und Hilfsstoffe kommen zum Beispiel solche Stoffe in Frage, die in folgenden Literaturstellen als Hilfsstoffe für Pharmazie, Kosmetik und angrenzende Gebiete empfohlen beziehungsweise angegeben sind: Ullmanns Enzyklopädie der technischen Chemie, Band 4 (1953), Seite 1 bis 39; Journal of Pharmaceutical Sciences, Band 52 (1963), Seite 918 ff ., H. v. Czetsch-Lindenwald, Hilfsstoffe für Pharmazie und angrenzende Gebiete; Pharm. Ind., Heft 2 (1961), Seite 72 ff.; Dr. H.P. Fiedler, Lexikon der Hilfsstoffe für Pharmazie, Kosmetik und angrenzende Gebiete, Cantor KG, Aulendorf in Württemberg (1989).

Die pharmazeutische und galenische Handhabung der R-beziehungsweise S-α-Liponsäure erfolgt nach den üblichen Standardmethoden. Beispielsweise wird R-beziehungsweise S-α-Liponsäure und Hilfsbeziehungsweise Trägerstoffe durch Rühren oder Homogenisieren (zum Beispiel mittels üblicher Mischgeräte) gut vermischt, wobei im allgemeinen bei Temperaturen zwischen 20 und 50^{o} C, vorzugsweise 20 bis 40^{o} C, Insbesondere bei Raumtemperatur gearbeitet wird. Im übrigen wird auf das folgende Standardwerk verwiesen: Sucker, Fuchs, Speiser, Pharmazeutische Technologie, Thieme-Verlag Stuttgart, 1978.

Die Applikation der R- beziehungsweise S-α-Liponsäure beziehungsweise der Arzneimittel kann auf die Haut oder Schleimhaut oder in das Körperinnere erfolgen, beispielsweise oral, enteral, pulmonal, nasal, lingual, intravenös, Intraarteriell, intrakardial, intramuskulär, intraperitoneal, intracutan, subcutan.

Bei den parenteralen Zubereitungsformen handelt es sich insbesondere um sterile beziehungsweise sterilisierte Erzeugnisse.

Falls die R- beziehungsweise S-α-Liponsäure in Form ihrer Salze verwendet wird, kann der Salzbildner auch Im Überschuß eingesetzt werden, das heißt in einer höheren Menge als äquimolar.

Beispiele für die Träger- und Hilfsstoffe sind Gelatine, natürliche Zucker wie Rohrzucker oder Milchzucker, Lecithin, Pektin, Stärke (zum Beispiel Maisstärke oder Amylose), Cyclodextrine und Cyclodextrinderivate, Dextran, Polyvinylpyrrolidon, Polyvinylacetat, Gummi arabicum, Alginsäure, Tylose, Talkum, Lycopodium, Kieselsäure (zum Beispiel kolloidale), Cellulose, Cellulosederivate (zum Beispiel Celluloseether, bei denen die Cellulose-Hydroxygruppen teilweise mit niederen gesättigten aliphatischen Alkoholen und/oder niederen gesättigten aliphatischen Oxyalkoholen verethert sind, zum Beispiel Methyloxypropylcellulose, Methylcellulose, Hydroxypropylmethylcellulose, Hydroxypropylmethylcellulosephthalat); Fettsäuren sowie Magnesium-, Calcium- oder Aluminiumsalze von Fettsäuren mit 12 bis 22 C-Atomen, insbesondere der gesättigten (zum Beispiel Stearate), Emulgatoren, Öle und Fette, Insbesondere pflanzliche (zum Beispiel Erdnußöl, Rizinusöl, Olivenöl, Sesamöl, Baumwollsaatöl, Maisöl, Weizenkeimöl, Sonnenblumensamenöl, Kabeljau-Leberöl, jeweils auch hydriert); Glycerinester und Polyglycerinester aus gesättigten Fettsäuren C₁₂H₂₄O₂ bis C₁₈H₃₆O₂ und deren Gemische, wobei die Glycerin-Hydroxygruppen vollständig oder auch nur teilweise verestert sind (zum Beispiel Mono-, Di- und Triglyceride);
pharmazeutisch verträgliche ein- oder mehrwertige Alkohole und Polyglykole wie Polyethylenglykole (Molekulargewichtsbereich zum Beispiel 300 bis 1500) sowie Derivate hiervon, Polyethylenoxid, Ester von aliphatischen gesättigten oder ungesättigten Fettsäuren (2 bis 22 C-Atome, insbesondere 10 - 18 C-Atome) mit einwertigen aliphatischen Alkoholen (1 bis 20 C-Atome) oder mehrwertigen Alkoholen wie Glykolen, Glycerin, Diethylenglykol, Pentaerythrit, Sorbit, Mannit und so weiter, die gegebenenfalls auch verethert sein können, Ester der Zitronensäure mit primären Alkoholen, Essigsäure, Harnstoff, Benzylbenzoat, Dioxolane, Glyzerinformale, Tetrahydrofurfurylalkohol, Polyglykolether mit C₁-C₁₂-Alkoholen, Dimethylacetamid, Lactamide, Lactate, Ethylcarbonate, Silicone (insbesondere mittelviskose Polydimethylsiloxane), Calciumcarbonat, Natriumcarbonat, Calciumphosphat, Natriumphosphat, Magnesiumcarbonat und ähnliche.

Als weitere Hilfsstoffe kommen auch Stoffe in Frage, die den Zerfall bewirken (sogenannte Sprengmittel) wie: quervernetztes Polyvinylpyrrolidon,
Natriumcarboxymethylstärke,
Natriumcarboxymethylcellulose oder mikrokristalline Cellulose. Ebenfalls können bekannte Hüllstoffe verwendet werden. Als solche kommen beispielsweise in Frage: Polymerisate sowie Copolymerisate der Acrylsäure und/oder Methacrylsäure und/oder deren Ester; Copolymerisate aus Acryl- und
Methacrylsäureestern mit einem geringen Gehalt an Ammoniumgruppen (zum Beispiel Eudragit® RS), Copolymerisate aus Acryl- und Methacrylsäureestern und Trimethylammoniummethacrylat (zum Beispiel Eudragit® RL); Polyvinylacetat; Fette, Öle, Wachse, Fettalkohole;
Hydroxypropylmethylcellulosephthalat oder -acetatsuccinat; Cellulose-acetatphthalat Stärke-acetatphthalat sowie Polyvinylacetatphthalat; Carboxymethylcellulose; Methylcellulosephthalat, Methylcellulosesuccinat, -phthalatsuccinat sowie Methylcellulose-phthalsäurehalbester; Zein; Ethylcellulose sowie Ethylcellulosesuccinat; Schellack, Gluten; Ethylcarboxyethylcellulose; Ethacrylat-Maleinsäureanhydrid-Copolymer; Maleinsäurenanhydrid-Vinylmethylether-Copolymer; Styrol-Maleinsäure-Copolymerisate; 2-Ethyl-hexyl-acrylatmaleinsäureanhydrid; Crotonsäure-Vinyl acetat-Copolymer; Glutaminsäure/Glutaminsäureester-Copolymer; Carboxymethylethyl-celluloseglycerinmonooctanoat; Celluloseacetatsuccinat; Polyarginin. als Plastifizierungsmittel für Hüllstoffe können in Frage kommen:
Citronen- und Weinsäureester (Acetyltriethylcitrat, Acetyltributyl-, Tributyl-, Triethyl-citrat); Glycerin und Glycerinester (Glycerindiacetat, -triacetat, acetylierte Monoglyceride, Rizinusöl); Phthalsäureester (Dibutyl-, Diamyl-, Diethyl-, Dimethyl-, Dipropyl-phthalat), Di-(2-Methoxy- oder 2-ethoxyethyl)-phthalat, Ethylphthalylglycolat, Butylphthalylethylglycolat und Butylglycolat; Alkohole (Propylenglycol, Polyethylenglycol verschiedener Kettenlängen), Adipate (Diethyl-adipat, Di-(2-Methoxy-oder 2-ethoxyethyl)-adipat); Benzophenon; Diethyl- und Dibutylsebacat, Dibutylsuccinat, Dibutyltartrat; Diethylenglycoldipropionat; Ethylenglykoldiacetat, -dibutyrat, -dipropionat; Tributylphosphat, Tributyrin; Polyethylenglykolsorbitanmonooleat (Polysorbate wie Polysorbat 80); Sorbitanmonooleat.

Zur Herstellung von Lösungen oder Suspensionen kommen beispielsweise Wasser oder physiologisch verträgliche organische Lösungsmittel in Frage, wie zum Beispiel Alkohole (Ethanol, Propanol, Isopropanol, 1,2-Propylenglykol, Polyglykole und deren Derivate, Fettalkohole, Partialester des Glycerins), Öle (zum Beispiel Erdnußöl, Olivenöl, Sesamöl, Mandelöl, Sonnenblumenöl, Sojabohnenöl, Ricinusöl, Rinderfußöl), Paraffine, Dimethylsulfoxid, Triglyceride und ähnliche.
Für injizierbare Lösungen oder Suspensionen kommen zum Beispiel nicht-toxische parenteral verträgliche Verdünnungsmittel oder Lösungsmittel in Frage, wie zum Beispiel : Wasser, 1,3-Butandiol, Ethanol, 1,2-Propylenglykol, Polyglykole in Mischung mit Wasser, Glycerol, Ringer's Lösung, isotonische Kochsalzlösung oder auch gehärtete Öle einschließlich synthetischer Mono- oder Diglyceride oder Fettsäuren wie Oleinsäure.

Bei der Herstellung der Zubereitungen können bekannte und übliche Lösungsvermittler, beziehungsweise Emulgatoren, verwendet werden. Als Lösungsvermittler und Emulgatoren kommen beispielsweise in Frage: Polyvinylpyrrolidon, Sorbitanfettsäureester wie Sorbitantrioleat, Phosphatide, wie Lecithin, Acacia, Traganth, polyoxyethyliertes Sorbitanmonooleat und andere ethoxylierte Fettsäureester des Sorbitan, polyoxyethylierte Fette, polyoxyethylierte Oleotriglyceride, linolisierte Oleotriglyceride, Polyethylenoxyd-Kondensationsprodukte von Fettalkoholen, Alkylphenolen oder Fettsäuren oder auch 1-Methyl-3-(2-hydroxyethyl)imidazolidon-(2).
Polyoxyethyliert bedeutet hierbei, daß die betreffenden Stoffe Polyoxyethylenketten enthalten, deren Polymerisationsgrad im allgemeinen zwischen 2 bis 40 und insbesondere zwischen 10 bis 20 liegt. Solche polyoxyethylierten Stoffe können beispielsweise durch Umsetzung von hydroxylgruppenhaltigen Verbindungen (beispielsweise Mono- oder Diglyceride oder ungesättigte Verbindungen wie zum Beispiel solchen, die Ölsäurereste enthalten) mit Ethylenoxyd erhalten werden (zum Beispiel 40 Mol Ethylenoxyd pro 1 Mol Glycerid).
Beispiele für Oleotriglyceride sind Olivenöl, Erdnußöl, Rizinusöl, Sesamöl, Baumwollsaatöl, Maisöl.

Siehe auch Dr. H. P. Fiedler "Lexikon der Hilfsstoffe für Pharmazie, Kosmetik und angrenzende Gebiete" 1971, S. 191-195.

Darüberhinaus ist der Zusatz von Konservierungsmitteln, Stabillsatoren, Puffersubstanzen, Geschmackskorrigentien, Süßmitteln, Farbstoffen, Antioxydantien und Komplexbildnern und dergleichen möglich.
Als Komplexbildner kommen beispielsweise in Frage: Chelatbildner wie Ethylendiamino-tetraessigsäure, Nitrilotriessigsäure, Diethylentriaminpentaessigsäure sowie deren Salze.
Weiterhin kommen als Komplexbildner auch solche in Frage, die R- oder S-α-Liponsäure in einem Hohlraum einschließen. Beispiele hierfür sind Harnstoff, Thioharnstoff, Cyclodextrine, Amylose.
Gegebenenfalls ist zur Stabilisierung des Wirkstoffmoleküls mit physiologisch verträglichen Basen oder Puffern auf einen pH-Bereich von ca. 6 bis 9 einzustellen. Im allgemeinen wird ein möglichst neutral er bis schwach basischer (bis pH 8) pH-Wert bevorzugt.

Als Antioxydantien kommen beispielsweise Natriumsulfit, Natriumhydrogensulfit, Natriummetabisulfit, Ascorbinsäure, Ascorbylpalmitat, -myristat, -stearat, Gallussäure, Gallussäure-alkylester, Butylhydroxyanisol, Nordihydroguajaretsäure, Tocopherole sowie Synergisten (Stoffe, die Schwermetalle durch Komplexbildung binden, beispielsweise Lecithin, Ascorbinsäure, Phosphorsäure Ethylendiaminotetraessigsäure, Citrate, Tartrate) zur Anwendung. Der Zusatz der Synergisten steigert die antioxygene Wirkung der Antioxydantien erheblich.
Als Konservierungsmittel kommen beispielsweise Sorbinsäure, p-Hydroxybenzoesäureester (zum Beispiel Niederalkylester), Benzoesäure, Natriumbenzoat, Trichlorisobutylalkohol, Phenol, Kresol, Benzethoniumchlorid, Chlorhexidin und Formalinderivate in Betracht.

Kurze Beschreibung der in der Anmeldung besonders erwähnten pharmakologischen Testmethoden:

### Randall-Selitto-Test (Entzündungsschmerz an der Ratte)

In Anlehnung an die Methode nach RANDALL und SELITTO (L.O. Randall u. J. Selitto, Arch. int. Pharmacodyn. Bd. 111, Seiten 409-418 (1957)) erhalten Ratten 0,1 ml einer 20%igen (in entmineralisiertem Wasser) Brauerhefesuspension in die rechte Hinterpfote subplantar injiziert. 2 1/2 Stunden danach werden Prüfsubstanzen verabreicht und 30 Minuten später die Schmerzschwelle mit einem im Handel befindlichen Algesiemeter als Druck (in Gramm) auf die entzündete Pfote gemessen. Als Kriterium gilt die Abwehrreaktion der Tiere, die Pfote wegzuziehen und/oder sich aus dem Griff des Experimentators zu befreien. Die Substanzwirkung aus der Erhöhung der Schmerzschwelle gegenüber einer unbehandelten Kontrollgruppe wird ermittelt. Der Versuchsablauf unterscheidet sich von der Originalmethode dadurch, daß die Substanzen erst 2 1/2 Stunden nach der Ödemsetzung und nicht mit dieser gleichzeitig verabreicht werden. Auf diese Weise soll verhindert werden, daß die Ödementwicklung durch eine mögliche antiphlogistische Wirkung gehemmt und die Analgesie überlagert, beziehungsweie vorgetäuscht wird.

Bestimmt wird die ED50 mittels der Methode der linearen Regression. Die ED50 ist hierbei die Dosis in mg/kg, bei der rechnerisch eine 50%ige analgetische Wirkung vorliegt.

### Essigsäure-Test (Writhing-Test) an der Maus

### Methode:

Im Essigsäure-Test nach KOSTER et al. (Fed. Proc., Bd. 18, Seite 412 (1959)) wird der Schmerzreiz durch eine intraperitoneale Injektion 1%iger Essigsäure ausgelöst. Die Schmerzreaktion äußert sich als charakteristisches Strecken der Tiere ("writhing syndrome"), das in unregelmäßigen Zeitabständen längere Zeit nach der Essigsäure-Injektion anhält. Die dosisabhängige Hemmung der Häufigkeit der Streckbewegung gegenüber einer unbehandelten Kontrollgruppe wird als analgetische Wirkung in Prozent ausgedrückt. Die Auswertung erfolgt durch Bestimmung der ED50 (Methode der linearen Regression). Die ED50 ist die Dosis in mg/kg, bei der eine 50%ige Hemmung des "writhing syndrome" vorliegt.

Der Essigsäure-Test zeichnet sich dadurch aus, daß nicht nur die Wirkung von starken, zentral wirksamen Analgetika, sondern auch von vorwiegend peripher wirksamen Analgetika-Antipyretika und antiphlogistisch wirksamen Pharmaka, wie Phenylbutazon, Indometacin u.a., nachweisbar ist Damit weist die Wirkung in der Versuchsanordnung auf eine periphere Komponente der Analgesie hin.

### Carrageenin-Ödem-Test auf antiphlogistische Wirkung

Die Untersuchung erfolgt am Carrageenin-Ödem der Rattenpfote in Anlehnung an die Methode von MÖRSDORF und Mitarbeiter (Arch.int.Pharmacodyn. 192, 111-127 (1971)). Die antiphlogistische Wirkung wird z.B. als Ödemhemmung in Prozent gegenüber der unbehandelten Kontrollgruppe angegeben. Es wird bei sämtlichen Versuchen oral oder intraperitoneal appliziert. Die Substanz wird 1 Stunde nach Auslösung der Entzündung oral oder intraperitoneal verabreicht. Die ED50 ist die Dosis in mg/kg, bei der eine 50%ige Hemmung des Pfotenödems vorliegt.

### Beispiele

### Beispiel 1: Tabletten mit 50 mg S- bzw. R-α-Liponsäure

250 g S-α-Llponsäure werden mit 750 g mikrokristalliner Cellulose gleichmäßig verrieben. Nach dem Sieben der Mischung werden 250 g Stärke (Starch 1500/Colorcon), 732,5 g Lactose, 15 g Magnesiumstearat und 2,5 g hochdisperses Siliciumdioxid zugemischt und die Mischung zu Tabletten vom Gewicht 400,0 mg verpreßt.

Eine Tablette enthält 50 mg S-α-Liponsäure.

In gleicher weise können Tabletten mit 50 mg R-α-Liponsäure hergestellt werden, wenn statt 250 g S-α-Li-ponsäure die gleiche Menge R-α-Liponsäure eingesetzt wird.

Gegebenenfalls können die Tabletten nach üblichen Verfahren mit einem magensaftlöslichen oder magensaftpermeablen Filmüberzug versehen werden.

### Beispiel 2: Ampullen mit 50 mg S- bzw. R-α-Liponsäure als Trometamolsalz in 2 ml

250 g S-α-Liponsäure werden zusammen mit 352,3 g Trometamol (2-Amino-2-(hydroxymethyl)-1,3-propandiol) in einer Mischung aus 9 Liter Wasser für Injektionszwecke und 200 g 1,2-Propylenglykol unter Rühren gelöst. Die Lösung wird mit Wasser für Injektionszwecke auf 10 Liter aufgefüllt und anschließend durch ein Membranfilter der Porenweite 0,2 µm mit Glas faservorfilter filtriert. Das Filtrat wird unter aseptischen Bedingungen zu 2 ml in sterilisierte 2-ml-Ampullen abgefüllt.

Eine Ampulle enthält in 2 ml Injektionslösung 50 mg S-α-Liponsäure als Trometamolsalz.

In gleicher Weise können Ampullen mit R-α-Liponsäure hergestellt werden, wenn statt 250 g S-α-Liponsäure die gleiche Menge R-α-Liponsäure eingesetzt wird.

## Patentansprüche

1. Verwendung von R-α-Liponsäure und deren pharmazeutisch verwendbaren Salze zur Herstellung eines Arzneimittels zur Zytoprotektion.

2. Verwendung von R-α-Liponsäure zur Herstellung eines Arzneimittels zur Zytoprotektion, dadurch gekennzeichnet, daß es neben der R-α-Liponsäure die üblichen pharmazeutischen Träger, Hilfsstoffe und/oder Verdünnungsstoffe enthält.

3. Verwendung von R-α-Liponsäure zur Herstellung eines Arzneimittels zur Zytoprotektion, dadurch gekennzeichnet, daß als Stabilisatoren beziehungsweise Lösungsvermittler folgende Stoffe verwendet werden:
aliphatische C₂-C₄-Alkohole, die eine, zwei oder drei Hydroxylgruppen enthalten, Polyethylenglykole mit Molgewichten zwischen 200 bis 600; übliche physiologische verträgliche organische Amide, natürliche α-Aminosäuren, aliphatische Amine, Hydroxyethyltheophyllin, Trometamol, Diethylenglykolmonomethylether.

4. Verwendung von R-α-Liponsäure zur Herstellung eines Arzneimittels zur Zytoprotektion, dadurch gekennzeichnet, daß die R-α-Liponsäure jeweils in einer Menge von 0,1 mg bis 6 g, vorzugsweise 0,1 bis 600 mg vorliegen.

5. Verfahren zur Herstellung eines Arzneimittels zur Zytoprotektion, dadurch gekennzeichnet, daß R-α-Liponsäure oder ein pharmazeutisch verwendbares Salz hiervon mit gebräuchlichen pharmazeutischen Trägerstoffen und/oder Verdünnungsmitteln beziehungsweise sonstigen Hilfsstoffen zu pharmazeutischen Zubereitungen verarbeitet beziehungsweise in eine therapeutisch anwendbare Form gebracht wird.

6. Verfahren nach Anspruch 5, dadurch gekennzeichnet, daß man R-α-Liponsäure oder ein pharmazeutisch verwendbares Salz hiervon zusammen mit üblichen Träger- und/oder Verdünnungs- beziehungsweise Hilfsstoffen bei Temperaturen zwischen 0 und 120 °C, vorzugsweise 20 bis 80 °C, vermischt beziehungsweise homogenisiert und gegebenenfalls die so erhaltene Mischung zur Herstellung von Zubereitungen, die in der Dosierungseinheit 0,1 mg bis 6 g, vorzugsweise 0,1 bis 600 mg R-α-Liponsäure oder ein pharmazeutisch verwendbares Salz enthalten, in Hohlzellen entsprechender Größe ausgießt, zu Tabletten verpreßt oder in Kapseln entsprechender Größe abfüllt oder granuliert und dann gegebenenfalls unter Zusatz von weiteren üblichen Hilfsstoffen zu Tabletten verpreßt oder in Kapseln abfüllt.

7. Verfahren nach Anspruch 5, dadurch gekennzeichnet, daß man R-α-Liponsäure oder ein pharmazeutisch verwendbares Salz hiervon bei Temperaturen zwischen 20 und 100 °C, gegebenenfalls mit 0,001 bis 1 Gewichtsteilen (bezogen auf 1 Gewichtsteil R-α-Liponsäure) Antioxidans sowie gegebenenfalls in Anwesenheit eines Komplexbildners und/oder eines Emulgators in Wasser, physiologisch unbedenklichen Alkoholen, Ölen oder Dimethylsulfoxid oder Mischungen hiervon auflöst oder suspendiert und gegebenenfalls die so erhaltene Lösung mit soviel Wasser, Alkohol, Dimethylsulfoxid oder Öl auffüllt, daß die resultierende Lösung, Suspension oder Emulsion 0,5 - 20 Gewichtsprozent an Wirkstoff R-α-Liponsäure enthält.
